# EUROPEAN PATENT SPECIFICATION

(11) **EP 3 377 227 B1**
(45) Date of publication and mention of the grant of the patent: **11.09.2019**
(21) Application number: 16801009.8
(22) Date of filing: 15.11.2016
(51) Int. Cl.: B01L 9/00, B01L 7/00

(54) **APPARATUS FOR CONDUCTING AN ASSAY**
VERFAHREN UND VORRICHTUNG ZUR DURCHFÜHRUNG EINES TESTS
APPAREIL DE RÉALISATION D'ESSAI

(30) Priority: 16.11.2015 GB 201520193
(43) Date of publication of application: 26.09.2018
(73) Proprietor: Mast Group Limited, Liverpool, Merseyside L20 1EA (GB)
(72) Inventor: WILLIAMS, David, Liverpool Merseyside L20 1EA (GB); ROSE, Howard, Liverpool Merseyside L20 1EA (GB); WONG, Ken, Liverpool Merseyside L20 1EA (GB)
(74) Representative: Rees, Kerry
(86) International application number: PCT/GB2016/053568
(87) International publication number: WO 2017/085472

(56) References cited:
- EP-A2- 0 947 819
- WO-A1-03/080868
- JP-A- H08 103 672
- US-A1- 2009 253 130
- US-A1- 2010 177 325
- US-A1- 2013 101 983

## Description

The present invention relates to an apparatus for conducting an assay, and particularly to an apparatus which controls the conditions to which so called "lab-on-a-disc" systems are subjected.

"Lab-on-a-disc" or "lab-on-a-CD" systems use generally disc-shaped reaction vessels containing passages, cavities and other features such as valves and zones of altered hydrophobicity. They are particularly useful for carrying out biological assays involving microfluidic transfers and reactions within the discs. The assay discs are used with apparatus which effects rotational movement to control the flow of reaction fluids using centrifugal forces.

Some known apparatuses for carrying out assays in assay discs comprise means for heating the environment around the assay disc so that the whole assay disc and its contents can be subjected to a particular temperature. Also known are assay discs which comprise heat elements which can bring about changes in temperature at particular locations of the assay discs, as in WO03/080868.

The present inventors have recognised shortcomings with known apparatuses and have developed a new system which allows more controlled assay disc procedures and brings further advantages.

From a first aspect the present invention provides a turntable for receiving an assay disc and to control an assay on said assay disc by rotational movement of said turntable, wherein said turntable comprises one or more heater modules to apply heat to one or more specific parts of said assay disc during rotation; a heater controller to select a required heater and to control the temperature thereof, and an IR transceiver to allow instructions and/or heating parameters to be transferred to the heater controller wirelessly.

The present invention allows much more specific control of heating than prior art apparatuses which heat up the environment around the assay disc and transfer heat by convection to the whole disc. In the present invention, the control is specific not only in terms of location to which the heat is applied, by conduction, but also in terms of the ramp up/ramp down time, i.e. the rate of change of temperature of reaction medium at a particular location on the disc.

The apparatus of the present invention can be configured so as to carry out isothermal assays (e.g. where the heater element is positioned substantially continuously across the whole area of the assay so that all of the assay is carried out at substantially the same temperature, or alternatively where no heat is applied). However, the apparatus of the present invention is perhaps even more useful when configured to carry out non-isothermal assays, i.e. where different parts of the assay disc, and therefore different parts of the reaction medium, are, or can be, subjected to different temperatures. The combination of centrifugal force and specific heating allows effective and tailorable operation. Unlike some prior art devices comprising heating elements within the assay discs, the heat comes from the turntable. Low amounts of power are required because the heat is specifically applied by conduction to specific parts of the assay disc, and the temperature can be adjusted quickly which can bring efficiencies not only in terms of the assay duration but also in terms of the amount of time it is necessary to use the heating elements.

During the assay, the assay disc stays fixed relative to the turntable. When placed on the turntable in a fixed position, one or more heaters on the turntable may align with one or more areas of the disc that may require controlled, localised heating.

Means may be provided to facilitate the correct positioning of the assay disc relative to the turntable. For example, the turntable may comprise a central spindle corresponding to a spindle hole in the assay disc. A longitudinal groove on the spindle and a corresponding longitudinal projection on the hub of the assay disc enable the angular position of the assay disc to be fixed relative to the turntable. Guiding means may be provided to guide the assay disc to the correct angular position. For example, the horizontal dimension of the groove of the spindle and the horizontal dimension of the complementary projection on the disc may decrease in a longitudinally downward direction: this means that when the disc is just above the turntable there is some tolerance in the angular positioning of the assay disc relative to the turntable, which tolerance decreases as the assay disc is lowered to the turntable.

Further means may be provided to locate the assay disc relative to the turntable. For example, a locating projection (e.g. a pin or boss) and corresponding locating recess (e.g. a hole or cut away portion) may be provided on the turntable and assay disc respectively, or vice versa. These locating features may be located at or near the periphery of the assay disc.

Thus there may be two or more means of locating the angular position of the assay disc relative to the turntable. For example, the central spindle locating means may act as a primary locator and guide means, and the projection and recess features at or near the periphery of the assay disc may act as more precise locating means.

Clamping means may be provided to secure the assay disc to the turntable.

These clamping means may comprise a mechanical ball bearing clamping mechanism to hold the assay disc to the turntable, for example at the central hub.

Alternatively or additionally the clamping means may comprise magnetic means for holding the assay disc in a fixed position relative to the turntable. A magnetic means may also be used to align and guide the disc to the correct position. Therefore, for example, a user may place the disc above the turntable and the apparatus will then automatically align the disc and securely hold it to the turntable in the correct position.

The magnetic means may comprise disc magnets arranged in opposite pole directions in the turntable (for example in the hub of the turntable) and disc magnets arranged in opposite pole directions in the assay disc (for example in the hub of the assay disc).

Wireless power transfer means may be used to transfer power to heater elements in the turntable and/or to a heater controller. One possible power transfer mechanism may comprise disc coils arranged in a concentric manner. The apparatus may comprise a primary disc coil and a secondary disc coil. For example, a primary disc coil (which may be fixed below the turntable) may be driven to generate an alternating electromagnetic field that will then induce current on a secondary disc coil (which may be fixed to the turntable and above the primary disc coil separated with a small gap).

The apparatus may also comprise an IR (infrared) transceiver for bi-directional communication. The IR bi-directional communication may be used to allow instructions and heating parameters to be transferred to a heater controller wirelessly and may also allow communication from the heater controller to the host by establishing handshake between the host and the heater controller. Each transceiver may optionally contain four IR emitters and one IR receiver. The four IR emitters may be arranged in equidistance surrounding the central hub of the turntable. The total field of emission from the four IR emitters allow the IR receiver at the opposite end to be able to receive the IR emitted signal across 360°, so that communication can be made regardless of the turntable position.

The host may communicate with the heater controller on the turntable via the IR bi-directional link. Accordingly, the host may instruct the heater controller to select a required heater and communicate a target temperature to the heater controller. The heater controller may then activate the heater.

Optionally, the heater controlled may be capable of continuously monitoring the temperature using a temperature sensor. Accordingly, the heater controller may be able to reach, and then maintain, a target temperature. Optionally, the sensing, reaching and maintenance of the target temperature may be achieved by the heater controller without receiving further instructions from the host.

The temperature of the heater may be controlled by varying the power to the heater. This may be achieved by any suitable means including, but not limited to, switvhing the power on and off, pulse width modulation of the power or analogue voltage, analogue current control, or any combination thereof.

Optionally, the host may be capable of selecting more than one heater and setting different target temperatures for each heater thus allowing different areas of the assay to be maintained at different temperatures. Alternatively, or in addition, the host may be capable of selecting more than one heater and setting different heating or cooling rates for each heater.

Optionally, the host may be capable of selecting more than one heater and setting different heating and or cooling programs for each heater, such that different areas of the assay may have their temperature varied according to different heating profiles.

Accordingly, the rate of temperature rise (or fall), i.e. the ramp-up (and ramp-down) time may be controlled by the host. This may be achieved by setting the controller target temperature to a temperature higher (or lower) than the current temperature. Optionally, the target temperature may be set to a temperature 1 degree higher (or lower) than the current temperature. Alternatively, the target temperature may be set to be more or less than one degree higher (or lower) than the current temperature. After a specific time delay, the target temperature may be incrementally increased (or decreased), with these steps repeated until a desired final temperature is reached.

Alternatively, the host may instruct the heater controller to perform the same task by communicating one or more of a start temperature, end temperature, temperature increment, temperature decrement amount and ramp rate to the heater controller.

In another variant, the or by the host may instruct the heater controller to perform the same task by communicating a series of ascending or descending target temperatures to the controller at fixed intervals.

The present invention thus provides heat to the assay disc whilst it is spinning. This heating can be provided in a continuous manner and/or the heating can be tailored.

A known problem which may occur in assay discs concerns the condition of the liquid when it is being heated. Bubbles may appear and/or there may be expansion of the air and/or pressure difference between chambers may have undesirable consequences, e.g. causing back-flow of liquid. The distorted condition of the liquid can affect the assay e.g. by affecting optical readout. The present invention brings advantages in restoring the original state of the liquid in the reaction wells through centrifugal rotation whilst heating the liquid. Thus the present invention avoids compromising the heating of the liquid, and consistent optical readout can be obtained.

The heater modules may for example be foil heaters.

The turntable may be mounted with one or more heater modules, optionally two or more heater modules.

Means to allow magnet transportation may be provided. An extraction process carried out on a sample in an assay disc may require extraction of nucleic acids (e.g. DNA or RNA) using magnetic bead transfer to obtain clean nucleic acid. For example, this may be done by using magnets (e.g. two magnets) positioned some distance beneath the turntable, which could be lifted up vertically, or by a magnet that could be controlled to move radially and lifted up vertically. When the transfer process is required, the magnets may be lifted up to contact with, or become closer to, the assay disc to attract the magnetic beads. A slot in the turntable may be provided to allow the two magnets to be lifted up without any obstacle in between the assay disc and the magnets. The slot could be positioned to align with the position of the two magnets by rotating the turntable. As regards a radial-movement - controlled magnet, the magnet may be moved along a path where the magnetic beads need to be transferred to.

The apparatus comprises circuitry for controlling a number of heater modules that could be controlled independently to heat up to the target temperature. The heaters may consist of a thin aluminium plate for evenly distributed heating across specific region(s) of the assay discs. An RTD (resistance temperature detector) may be mounted on the thin aluminium plate to monitor the temperature of the aluminium plate. The RTD may be connected to the heater controller circuit to provide feedback to regulate the heater to achieve stable temperature over time.

The wireless functionality may be present in terms of the power generation in order to power the circuitry and the heaters, and wireless communication may also be used with 360° IR transceiver configuration. The wireless features allow the turntable to be spun freely; at the same time the power can be supplied to operate the heaters and the IR communications.

The heater modules may be configured to protrude from the surface of the turntable using light spring pressure so as to be in contact with the assay disc so that heat can be transferred effectively.

Optionally, insulators may be used beneath the heater modules to prevent heat loss downwards. As a result they can improve the efficiency of heat transfer and the control of the heating rate.

In one embodiment there are two heater modules on the turntable: one may be used for heating up the sample in the sample chamber; and the other may be used for the reaction wells for an assay. Additional heater modules may be added for other heating requirements: for example, an additional heater could be used to assist fluid transfer by generating differential pressure that would allow the fluid to transfer from a heated area to a cold region.

A primary coil assembly may contain a primary coil attached to a ferrite sheet sandwiched between two holders which may conveniently be made of plastic. The same configuration may be used for a secondary coil assembly. The primary and secondary coil configurations may be used to provide the power to the heaters. The generated power (e.g. 5 Watts and above) could be customised according to the application needs by changing the configurations of the primary and secondary coils, and/or the signal frequency supplied to the primary coil. For example, the power generated and delivered to the heater and controller could be adjusted to between 5 Watts and 7 Watts by adjusting the frequency of signals supplied to primary coil. The power may be used to drive the heaters and the associated circuitry including the IR transceiver and the heater controller. In a further example, the power may be configured to be distributed between heaters, e.g. to a 4 Watt heater and a 2 Watt heater operating at the same time.

The whole turntable assembly may be attached to the spindle of a motor to establish the rotational movement on the turntable for controlling the movement of the fluid. In this example, a BLDC (Brushless DC) motor may be used. The motor may be mounted on a metal base (motor mount) which is fixed to the chassis. On top of the motor mount, the primary IR (Infrared) transceiver board may be mounted and followed by the primary coil. Both components may be mounted so that they are held stationary. Above the primary coil are the secondary coil and the circuit board for the secondary IR transceiver and heater controller. They may be mounted to the turntable. They could be spun along with the turntable without interrupting the power supply and IR communication between the host and heater controller.

Optionally, the apparatus of the present invention may comprise, in addition to the heater module(s) on the turntable, means for heating and/or cooling the ambient temperature of the chamber within which the assay disc is located during the assay. Thus, whereas the primary heating system provides localised heating from the turntable, an optional secondary heating system allows the temperature of the environment around the assay disc (and, consequently, the assay disc and its contents) to be altered. Optionally a fan heater or cooler may be used. For example, an air blower, using a fan, may be used.

The present invention will now be described in further, non-limiting, detail, with reference to the following Examples and Figures in which:
Figure 1 shows a schematic representation of one example of a turntable platter in accordance with the present invention;
Figure 2 shows how magnetic locating and clamping means may be used in the present invention;
Figure 3 shows a side view of a turntable in accordance with the present invention;
Figure 4 shows a perspective view of a turntable in accordance with the present invention;
Figure 5 shows, in exploded schematic view, some possible elements of a turntable in accordance with the present invention; and
Figures 6 to 11 show some components of a turntable in accordance with the present invention.

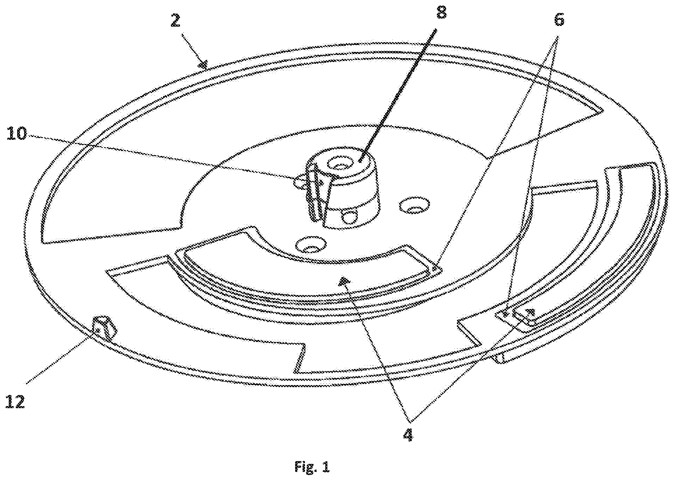

With reference to Figure 1, turntable platter 2 comprises heaters 4 and insulators 6. The insulators 6 are inserted in between the heaters 4 and the turntable and reduce the heat loss due to conduction. As a result, they improve heat efficiency and heat rate.

The turntable platter 2 comprises a central spindle 8 which itself comprises a longitudinal groove 10 which functions as an inner guide feature to allow coarse alignment of an assay disc 100 onto the spindle 8. Turntable platter 2 comprises boss 12 which acts as a finer alignment feature.

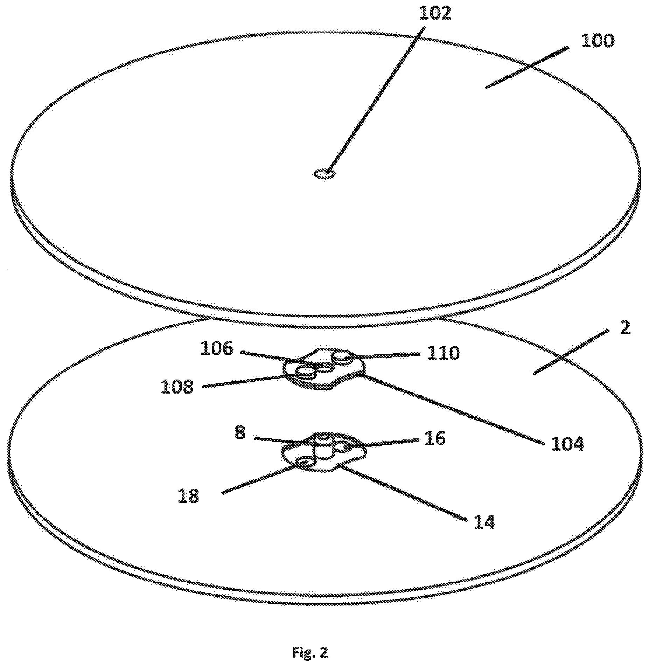

The magnetic means of locating and securing the assay disc 100 to the turntable platter 2 are shown most clearly in Figure 2. Most of the features of the turntable platter 2 and the assay disc 100 are omitted from this Figure for clarity. Assay disc 100 comprises spindle hole 102 for locating on spindle 18 of turntable platter 2. The turntable comprises disc magnet assembly 14 with north pole 16 and south pole 18. Assay disc 100 comprises corresponding disc magnetic assembly 104 consists of a north pole disc magnet 108 and a south pole disc magnet 110, which for clarity is shown separate from assay disc 100 in Figure 2, though in use it is fixed to the assay disc 100, for example by using a neoprene sheet. The neoprene sheet may be pre-cut to a shape as shown in Figure 2 that only allows the assay disc 100 to be sited on the turntable in a specific orientation. The neoprene sheet also provides a frictional grip that prevents the assay disc 100 from skidding when it is spun to high speed.

The magnet assemblies 14 and 104 are conveniently positioned at the hub of the apparatus, and accordingly in the embodiment shown, this magnet assembly 104 has hole 106 corresponding to spindle hole 102 of the assay disc.

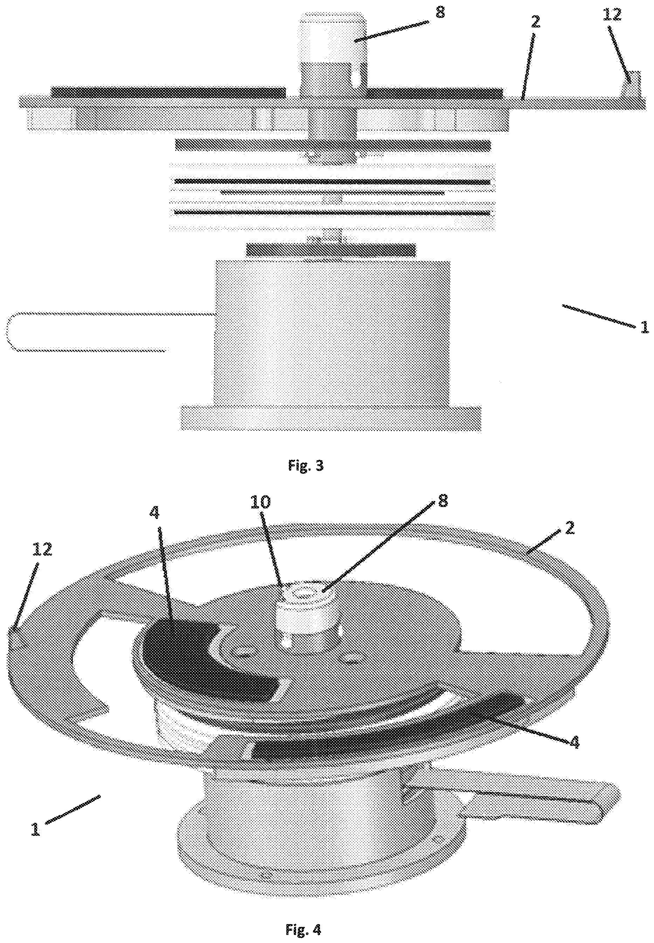

A side view and a perspective view of a turntable in accordance with the present invention are shown in Figures 3 and 4 respectively.

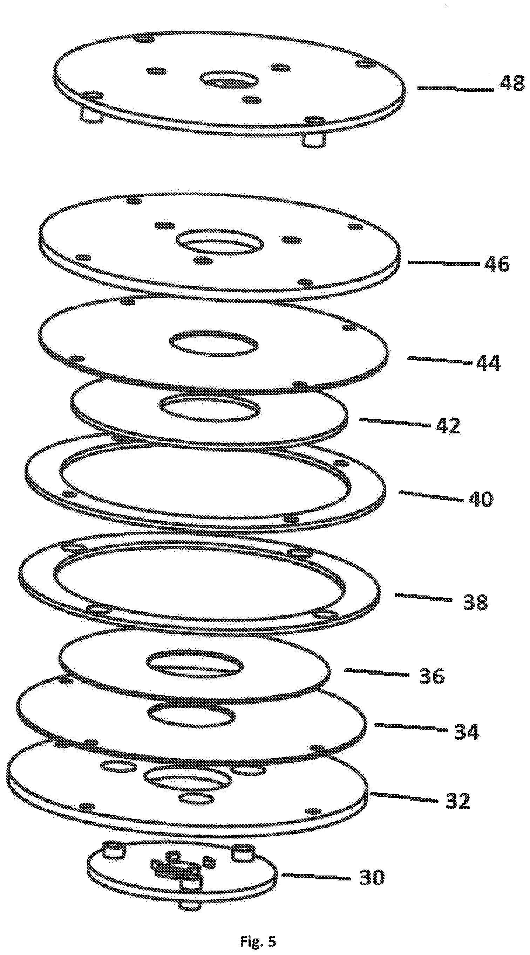

With reference to Figure 5, turntable 1 may comprise primary infrared transceiver 30, primary coil lower holder 32, ferrite sheet 34, primary coil 36, primary coil upper holder 38, secondary coil lower holder 40, secondary coil 42, ferrite sheet 44, secondary coil upper holder 46 and assembly 48 comprising a heater controller and a secondary infrared transceiver. The lower and upper holders for both the primary coil and the secondary coil act as clamps and may conveniently be made from plastic material.

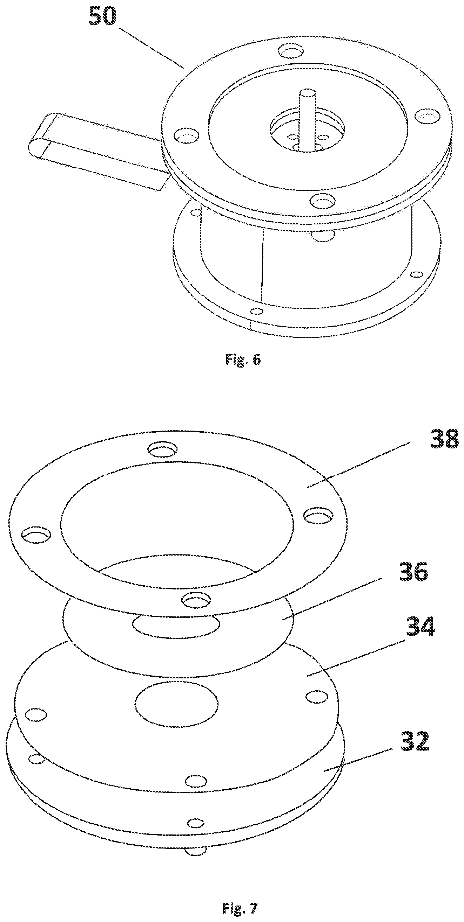

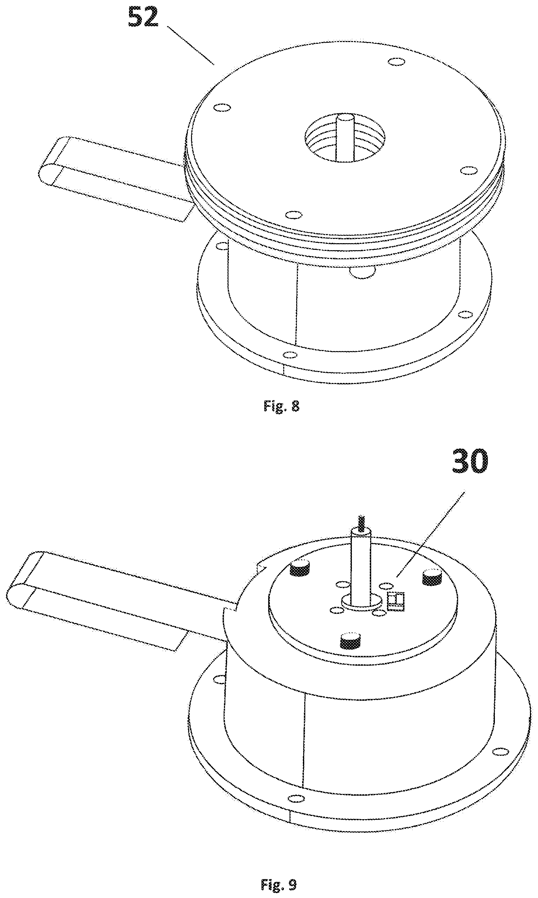

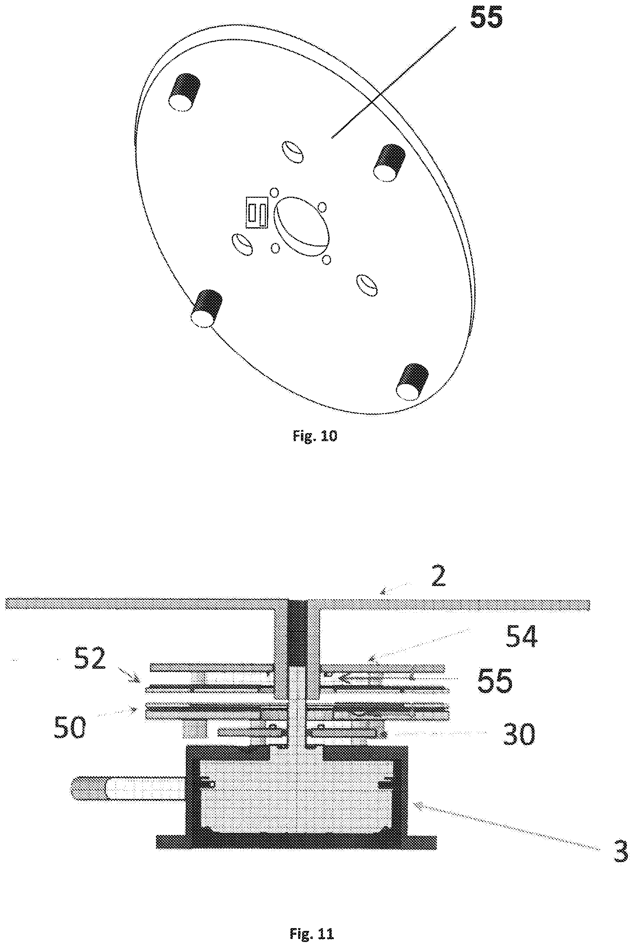

Some of the components of the turntable are shown in Figures 6 to 11. A schematic perspective view of the primary coil assembly 50 is shown in exploded view in relation to some of the components in Figure 7, and Figure 8 shows the secondary coil assembly 52 positioned on top of the primary coil assembly 50. IR transceiver primary and secondary components 30, 55 are shown in Figures 9 and 10 respectively. A side view showing the full construction of a turntable assembly 1 is shown in Figure 11. It starts with the BLDC motor mount component 3 as the base. The first component sitting on top of the BLDC mount is the primary IR transceiver component 30. It is followed by the primary coil component 50. Beneath turntable assembly component 2 are secondary IR transceiver component 55 and heater controller circuitry component 54. Components 55 and 54 share the same PCB board.

## Claims

1. Turntable for receiving an assay disc and to control an assay on said assay disc by rotational movement of said turntable, wherein said turntable comprises:
one or more heater modules to apply heat to one or more specific parts of said assay disc during rotation;
a heater controller to select a required heater and to control the temperature thereof, and
an IR transceiver to allow instructions and/or heating parameters to be transferred to the heater controller wirelessly.

2. Turntable as claimed in claim 1 further comprising means to facilitate the correct positioning of the assay disc relative to the turntable.

3. Turntable as claimed in claim 2 wherein said means comprise a central spindle on the turntable corresponding to a spindle hole in the assay disc, and wherein said spindle has a longitudinal groove to correspond with a longitudinal projection on the hub of the assay disc and optionally wherein the horizontal dimension of the groove of the spindle decreases in a longitudinally downward direction.

4. Turntable as claimed in claim 2 wherein said means comprise a locating projection on the turntable to mate with a corresponding locating recess on an assay disc, optionally at, adjacent to, or near the periphery of the assay disc.

5. Turntable as claimed in claim 2 wherein means of locating the assay disc relative to the turntable are as claimed in claim 3 and also as claimed in claim 4.

6. Turntable as claimed in any preceding claim further comprising clamping means to secure the assay disc to the turntable, optionally wherein said clamping means comprise a mechanical ball bearing clamping mechanism to hold the assay disc to the turntable, and/or further optionally wherein said clamping means comprise magnetic means for holding the assay disc in a fixed position relative to the turntable and/or for aligning and/or guiding the disc to the correct position.

7. Turntable as claimed in claim 6 wherein said magnetic means comprise disc magnets arranged in opposite pole directions in the turntable corresponding to disc magnets arranged in opposite pole directions in the assay disc.

8. Turntable as claimed in any preceding claim further comprising wireless power transfer means to transfer power to heater modules in the turntable and/or to the heater controller.

9. Turntable as claimed in any preceding claim further wherein the IR transceiver comprises four IR emitters and one IR receiver, and optionally wherein the four emitters are arranged in equidistance surrounding the central hub of the turntable.

10. Turntable as claimed in any preceding claim wherein there are two or more heater modules and/or optionally wherein the heater modules are independently controlled.

11. Turntable as claimed in any of claims 9 to 10 wherein the power supplied to the heater can be shared and distributed among the heaters on demand.

12. Turntable as claimed in any preceding claim comprising a primary coil assembly and a secondary coil assembly.

13. Assay unit comprising a turntable as claimed in any preceding claim, optionally the assay unit further comprises means for heating and/or cooling the ambient temperature of the chamber within which the assay disc is located during the assay, and further optionally wherein said means is a fan heater or cooler.

14. The combination of a turntable as claimed in any of claims 1 to 11 or an assay unit as claimed in claim 13, and an assay disc configured to be located on the turntable.

15. Use of an apparatus as claimed in any preceding claim to conduct an assay.

## Patentansprüche

1. Drehtisch zum Aufnehmen einer Assay Disc und zum Steuern eines Assay auf der Assay Disc durch Drehbewegung des Drehtisches, wobei der Drehtisch Folgendes umfasst:
ein oder mehrere Heizungsmodule zum Aufbringen von Wärme auf einen oder mehrere bestimmte Teile der Assay Disc während der Drehung;
eine Heizungsregler zum Auswählen einer benötigten Heizung und zum Regeln der Temperatur derselben, und
eine IR-Sende-Empfangsvorrichtung um die drahtlose Übertragung von Anweisungen und/oder Heizungsparametern an den Heizungsregler zu ermöglichen.

2. Drehtisch nach Anspruch 1, weiter umfassend Mittel zum Erleichtern der korrekten Positionierung der Assay Disc relativ zu dem Drehtisch.

3. Drehtisch nach Anspruch 2, wobei die Mittel eine zentrale Spindel an dem Drehtisch umfassen, die einem Spindelloch in der Assay Disc entspricht, und wobei die Spindel eine längslaufende Rille aufweist, um einem längslaufenden Vorsprung an der Nabe der Assay Disc zu entsprechen, und wobei optional das Horizontalmaß der Rille der Spindel in einer in Längsrichtung nach unten gehenden Richtung abnimmt.

4. Drehtisch nach Anspruch 2, wobei die Mittel einen Fixierungsvorsprung an dem Drehtisch umfassen, um mit einer entsprechenden Fixierungsaussparung an einer Assay Disc, optional an dem, benachbart dem oder in der Nähe des Rands der Assay Disc, zusammenzupassen.

5. Drehtisch nach Anspruch 2, wobei Mittel zum Fixieren der Assay Disc relativ zu dem Drehtisch wie in Anspruch 3 beansprucht und außerdem wie in Anspruch 4 beansprucht sind.

6. Drehtisch nach einem der vorangehenden Ansprüche, weiter umfassend Klemmmittel zum Befestigen der Assay Disc an dem Drehtisch, wobei optional die Klemmmittel einen mechanischen Kugellager-Klemmmechanismus umfassen, um die Assay Disc an dem Drehtisch zu halten, und/oder wobei ferner optional die Klemmmittel magnetische Mittel umfassen, um die Assay Disc in einer festen Lage relativ zu dem Drehtisch halten und/oder um die Disc in der korrekten Lage auszurichten und/oder sie in die korrekte Lage zu führen.

7. Drehtisch nach Anspruch 6, wobei die magnetischen Mittel Scheibenmagnete umfassen, die in entgegengesetzten Polrichtungen in dem Drehtisch angeordnet sind und in entgegengesetzten Polrichtungen in der Assay Disc angeordneten Scheibenmagneten entsprechen.

8. Drehtisch nach einem der vorangehenden Ansprüche, weiter umfassend drahtlose Energieübertragungsmittel zum Übertragen von Energie an Heizungsmodule in dem Drehtisch und/oder an den Heizungsregler.

9. Drehtisch nach einem der vorangehenden Ansprüche, wobei ferner die IR-Sende-Empfangsvorrichtung vier IR-Emitter und einen IR-Empfänger umfasst, und wobei optional die vier Emitter in gleichen Abständen die zentrale Nabe des Drehtischs umgebend angeordnet sind.

10. Drehtisch nach einem der vorangehenden Ansprüche, wobei zwei oder mehr Heizungsmodule vorgesehen sind und/oder wobei optional die Heizungsmodule unabhängig geregelt werden.

11. Drehtisch nach einem der Ansprüche 9 bis 10, wobei die der Heizung zugeführte Energie nach Bedarf zwischen den Heizungen aufgeteilt und verteilt werden kann.

12. Drehtisch nach einem der vorangehenden Ansprüche, umfassend eine Primärspulenanordnung und eine Sekundärspulenanordnung.

13. Assay-Einheit, umfassend einen Drehtisch nach einem der vorangehenden Ansprüche, wobei optional die Assay-Einheit weiter Mittel zum Erwärmen und/oder Abkühlen der Umgebungstemperatur der Kammer, in der sich die Assay Disc während des Assays befindet, umfasst, und wobei es sich ferner optional bei den Mitteln um einen Heizlüfter oder Kühllüfter handelt.

14. Kombination aus einem Drehtisch nach einem der Ansprüche 1 bis 11 oder einer Assay-Einheit nach Anspruch 13 und einer Assay Disc, die dazu konfiguriert ist, auf dem Drehtisch fixiert zu werden.

15. Verwendung einer Vorrichtung nach einem der vorangehenden Ansprüche, um einen Assay auszuführen.

## Revendications

1. Plateau tournant destiné à recevoir un disque de dosage et à commander un dosage sur ledit disque de dosage par le mouvement rotatif dudit plateau tournant, dans lequel ledit plateau tournant comprend : un ou plusieurs module de chauffage pour appliquer de la chaleur à une ou plusieurs parties spécifiques dudit disque de dosage pendant la rotation ;
un dispositif de commande de chauffage pour sélectionner un chauffage requis et pour commander la température de celui-ci, et
un émetteur-récepteur à infrarouge pour permettre le transfert d'instructions et/ou de paramètres de chauffage au dispositif de commande de chauffage sans fil.

2. Plateau tournant selon la revendication 1 comprenant en outre un moyen pour faciliter le positionnement correct du disque de dosage par rapport au plateau tournant.

3. Plateau tournant selon la revendication 2 dans lequel ledit moyen comprend un axe central sur le plateau tournant correspondant à un alésage d'axe dans le disque de dosage, et dans lequel ledit axe a une rainure longitudinale pour correspondre à une partie saillante longitudinale sur le moyeu du disque de dosage et facultativement dans lequel la dimension horizontale de la rainure de l'axe diminue dans une direction vers le bas longitudinalement.

4. Plateau tournant selon la revendication 2 dans lequel ledit moyen comprend une partie saillante de positionnement sur le plateau tournant pour s'accoupler avec un évidemment de positionnement correspondant sur un disque de dosage, facultativement au niveau, adjacent à, ou proche de la périphérie du disque de dosage.

5. Plateau tournant selon la revendication 2 dans lequel le moyen de positionnement du disque de dosage par rapport au plateau tournant est selon la revendication 3 et aussi selon la revendication 4.

6. Plateau tournant selon l'une quelconque des revendications précédentes comprenant en outre un moyen de serrage pour fixer le disque de dosage au plateau tournant, facultativement dans lequel ledit moyen de serrage comprend un mécanisme de serrage à roulement à billes mécanique pour maintenir le disque de dosage sur le plateau tournant, et/ou en outre facultativement dans lequel ledit moyen de serrage comprend un moyen magnétique destiné à maintenir le disque de dosage dans une position fixe par rapport au plateau tournant et/ou à aligner et/ou à guider le disque vers la position correcte.

7. Plateau tournant selon la revendication 6 dans lequel ledit moyen magnétique comprend des aimants de disque agencés dans des directions de pôles opposés dans le plateau tournant correspondant aux aimants de disque agencés dans des directions de pôles opposés dans le disque de dosage.

8. Plateau tournant selon l'une quelconque des revendications précédentes comprenant en outre un moyen de transfert de puissance sans fil pour transférer de la puissance à des modules de chauffage dans le plateau tournant et/ou au dispositif de commande de chauffage.

9. Plateau tournant selon l'une quelconque des revendications précédentes dans lequel en outre l'émetteur-récepteur à infrarouge comprend quatre émetteurs à infrarouge et un récepteur à infrarouge, et facultativement dans lequel les quatre émetteurs sont agencés à équidistance entourant le moyeu central du plateau tournant.

10. Plateau tournant selon l'une quelconque des revendications précédentes dans lequel il y a deux modules de chauffage ou plus et/ou facultativement dans lequel les modules de chauffage sont commandés indépendamment.

11. Plateau tournant selon l'une quelconque des revendications 9 à 10 dans lequel la puissance alimentant le chauffage peut être partagée et répartie entre les chauffage à la demande.

12. Plateau tournant selon l'une quelconque des revendications précédentes comprenant un ensemble de bobine primaire et un ensemble de bobine secondaire.

13. Unité de dosage comprenant un plateau tournant selon l'une quelconque des revendications précédentes, facultativement l'unité de dosage comprend en outre un moyen destiné à chauffer et/ou à refroidir la température ambiante de la chambre au sein de laquelle le disque de dosage est positionné pendant le dosage, et en outre facultativement dans laquelle ledit moyen est un chauffage ou un refroidisseur à ventilateur.

14. Combinaison d'un plateau tournant selon l'une quelconque des revendications 1 à 11 ou d'une unité de dosage selon la revendication 13, et d'un disque de dosage configuré pour être positionné sur le plateau tournant.

15. Utilisation d'un appareil selon l'une quelconque des revendications précédentes pour mener un dosage.
